# FASCICULE DE BREVET EUROPEEN

(11) **EP 0 822 976 B1**
(45) Date de publication et mention de la délivrance du brevet: **17.05.2000**
(21) Numéro de dépôt: 96910867.9
(22) Date de dépôt: 22.04.1996
(51) Int. Cl.: C12M 3/06

(54) **BIOREACTEUR**
BIOREAKTOR
BIOREACTOR

(30) Priorité: 28.04.1995 BE 9500391
(43) Date de publication de la demande: 11.02.1998
(73) Titulaire: ORGANOGENESIS INC., Canton, MA 02021 (US)
(72) Inventeur: KERSTEN, Jean, 7812 Villers-Saint-Amand (BE); BADER, Augustinus, 31275 Lehrte-Immensen (DE)
(74) Mandataire: Powis de Tenbossche, Roland
(86) Numéro de dépôt international: BE9600045
(87) Numéro de publication internationale: WO9634087

(56) Documents cités:
- EP-A- 0 113 328
- EP-A- 0 363 262
- EP-A- 0 419 234
- WO-A-85/01062
- WO-A-89/00188
- WO-A-90/13639
- WO-A-91/15570
- WO-A-93/18133
- DE-A- 4 230 194
- US-A- 3 948 732

## Description

### Abrégé de la divulgation

L'invention concerne un bioréacteur pour le traitement d'un fluide par des cellules. Ledit bioréacteur comporte un élément définissant une chambre dans laquelle sont situées des cellules pour le traitement du fluide, une membrane perméable aux liquides séparant ladite chambre d'un premier canal dans lequel s'écoule le fluide à traiter, et une membrane perméable aux gaz séparant ladite chambre d'un second canal dans lequel s'écoule un gaz contenant de l'oxygène.

### La technique antérieure

Des bioréacteurs en vue de la fixation de cellules sont construits à ce jour autour de faisceaux de fibres creuses comportant des cellules à l'intérieur ou à l'extérieur des fibres, ou comportant un système de culture de masse à l'intérieur ou à l'extérieur des fibres. Les cellules peuvent être encapsulées dans une matrice polymère comme du collagène, de l'alginate, etc.

Tous les bioréacteurs connus souffrent du fait que les cellules qui se fixent, et en particulier des hépatocytes, exigent une contiguïté et une monocouche pour réaliser une fonction métabolique équivalente à celle in vivo.

En outre, dans tous les systèmes des bioréacteurs connus, le fuide est d'abord oxygéné, ledit fluide oxygéné étant ensuite traité par les cellules.

On connaît par le document EP-A-0363262 un bioréacteur comportant un canal pour l'écoulement d'un fluide à traiter, un canal pour l'écoulement d'oxygène, une chambre de cultures dans laquelle passe une suspension de cellules, une membreane perméable au gaz séparant la chambre du canal d'écoulement d'oxygène et une membrane perméable au liquide séparant la chambre du canal pour l'écoulement du fluide.

Le bioréacteur de l'invention est destiné à résoudre ces problèmes. En outre, le bioréacteur de l'invention convient pour traiter des fluides par des cellules avec ou sans recouvrement par du collagène.

### Brève description de l'invention

La présente invention concerne un bioréacteur pour un traitement par cellules d'un milieu, ledit bioréacteur comportant :
(a) un premier canal pour l'écoulement dudit milieu à traiter ;
(b) un second canal pour l'écoulement d'un gaz contenant de l'oxygène ;
(c) un élément définissant une chambre contenant au moins deux couches de cellules pour traiter le milieu, lesdites couches étant séparées l'une de l'autre par une couche de collagène ;
(d) une membrane perméable au gaz séparant ladite chambre du second canal dans lequel s'écoule le gaz contenant de l'oxygène, et
(e) un film séparant ladite chambre dudit premier canal, ledit film étant constitué de deux membranes perméables aux liquides contiguës, une première membrane présentant des pores ou des ouvertures de diamètre supérieur à 3 µm ou d'environ 3 µm, tandis que la deuxième membrane présente des pores ou des ouvertures de diamètre inférieur à 3 µm.

Selon un mode de réalisation, le bioréacteur comporte deux membranes contiguës perméables aux liquides, une première présentant des pores ou des ouvertures d'un diamètre supérieur à 3 µm, tandis que la seconde présence des pores ou ouvertures d'un diamètre inférieur à 0,5 µm, en particulier inférieur à 0,3 µm.

Ce mode de réalisation convient particulièrement lorsque les cellules de chacune des couches de cellules utilisées pour le traitement sont incorporées dans une matrice de collagène ou sont piégées entre deux couches de collagène.

La membrane perméable aux liquides présente par exemple des caractéristiques hydrophobes ou une combinaison de caractéristiques hydrophobes et hydrophiles.

Selon un mode de réalisation préféré, le bioréacteur comporte une chambre contenant des cellules, ladite chambre communiquant avec le canal dans lequel s'écoule le fluide à traiter par l'intermédiaire d'une membrane perméable aux liquides, et avec le canal dans lequel s'écoule un gaz contenant de l'oxygène par l'intermédiaire d'une membrane perméable aux gaz, ladite chambre étant dotée d'une sortie de manière à extraire du fluide passant à travers la membrane perméable aux liquides en provenance du canal dans lequel s'écoule le milieu liquide à traiter. Cette sortie convient très bien pour extraire un composé biliaire de la chambre, de manière à maintenir un meilleur fonctionnement des cellules dans ladite chambre.

La chambre comporte au moins deux couches de cellules, lesdites couches étant de préférence séparées l'une de l'autre. Une co-culture est donc possible dans la chambre du bioréacteur. On peut par exemple utiliser dans la même chambre des cellules de Kupfer et des hépatocytes.

Par exemple, une première couche de cellules contient une proportion principale de cellules de Kupfer (par exemple une couche contiguë au canal d'écoulement du fluide à traiter) ou des cellules convenant pour fixer des toxines, alors que la seconde couche contient une proportion principale de hépatocytes.

Selon un autre mode de réalisation, la chambre comporte une entrée et une sortie de manière à permettre un écoulement du fluide contenant les cellules.

Selon un mode de réalisation, le bioréacteur comporte plusieurs unités empilées constituées chacune d'un cadre doté de deux couches, de manière à définir entre elles un canal pour l'écoulement d'un milieu liquide ou d'un gaz, une desdites couches étant une membrane perméable aux liquides ou une membrane perméable aux gaz. Une cavité ou passage est de préférence définie par les bords du cadre ou entre ces bords, une face de ladite cavité ou dudit passage étant l'une desdites couches, de préférence une membrane perméable aux liquides.

Selon un autre mode de réalisation, le bioréacteur comporte des unités empilées constituées chacune d'un cadre doté d'une couche de base, de manière à définir une cavité destinée à contenir les cellules prévues pour le traitement. Des tubes ou fibres peuvent s'étendre à l'intérieur de la cavité et présenter des caractéristiques de perméabilité aux liquides ou aux gaz.

L'invention concerne également:
* des unités de bioréacteur selon l'invention;
* un système de bioréacteur comportant un bioréacteur tel que revendiqué, ledit bioréacteur présentant une entrée pour l'introduction du fluide à traiter dans le canal à liquide, une sortie pour le fluide s'écoulant hors dudit canal à liquide, une entrée d'écoulement de gaz dans le bioréacteur, une sortie pour le gaz s'écoulant hors du bioréacteur et une sortie de collecte du liquide s'écoulant hors de la chambre, le système étant en outre doté de moyens de filtration du liquide s'écoulant hors de la chambre;
* des procédés (tels que des procédés de culture de cellules, des procédés de differentiation de cellules) utilisant un bioréacteur selon l'invention, et
* un support pour foie (foie artificiel ou dispostif pour faciliter le fonctionnement d'un foie défaillant).

Des avantages principaux du mode de réalisation du bioréacteur selon l'invention sont entre autres que:
* le géométrie du bioréacteur est définie de manière précise et permet un meilleur contrôle des transferts de masse;
* lorsqu'elles sont utilisées pour traiter un fluide, les cellules sont oxygénées;
* sa construction est facile et simple;
* il permet d'utiliser du collagène aussi bien que d'autres supports ou matrices;
* il existe la possibilité de traiter des produits biliaires et d'évacuer de la chambre un produit biliaire.

### Brève description des dessins

- La figure 1: est une vue partielle en perspective avec des coupes transversales d'un bioréacteur selon l'invention;
- La figure 2: est une vue avec des coupes transversales d'une unité du bioréacteur de la figure 1;
- Les figures 3 à 5: sont des vues d'autres modes de réalisation des unités;
- la figure 6: est une vue partielle en perspective avec une coupe transversale d'un système de bioréacteur selon l'invention;
- la figure 7: est une vue en coupe suivant la ligne VII-VII de la figure 6;
- la figure 8: est une vue éclatée d'un autre mode de réalisation d'une unité selon l'invention;
- la figure 9: est une vue en coupe transversale d'encore un autre mode de réalisation d'une unité selon l'invention;
- la figure 10: est une vue éclatée d'un autre mode de réalisation d'une unité selon l'invention;
- les figures 11 et 12: sont des vues en coupe transversale du mode de réalisation de la figure 10, le long des lignes XI-XI et XII-XII;
- la figure 13: est un graphique représentant la sécrétion d'albumine d'hépatocytes primaires cultivés en présence d'un gaz contenant 10% d'oxygène (en volumes ;
- la figure 14: est une vue d'un bioréacteur préféré selon l'invention; et
- la figure 15: est une vue schématique d'un système selon l'invention.

### Description de modes de réalisation selon l'invention

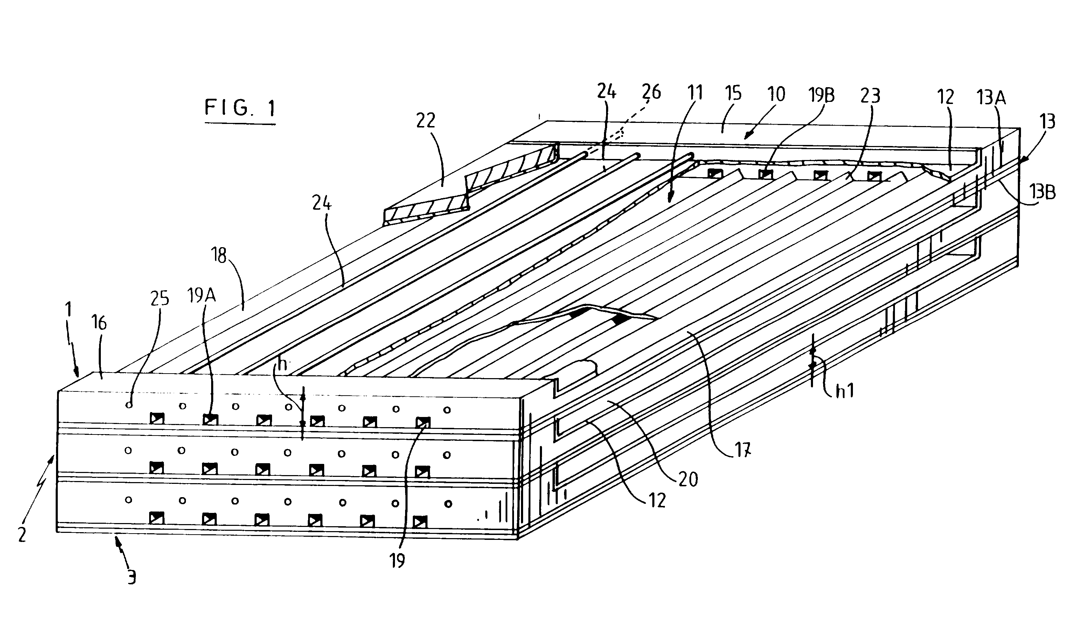

Le bioréacteur de la figure 1 pour un traitement par cellules d'un fluide comporte plusieurs unités de traitement 1, 2, 3, ... qui sont empilées ensemble.

Chaque unité est constituée d'un cadre 10 qui est doté sur les deux côtés d'un film perméable aux liquides 12, 13, par exemple un film présentant une porosité de moins de 3 micromètres, et de préférence de moins de 1 micromètre (par exemple 0,45 µm). Une chambre ou canal 11 est ainsi définie entre les deux films poreux du cadre.

Le cadre est constitué de quatre barres 15, 16, 17, 18, deux barres opposées 15, 16 présentant une hauteur ou épaisseur h supérieure à l'épaisseur h1 des deux autres barres 17, 18, ces dernières reliant chacune une extrémité de la barre 15 à une extrémité de la barre 16.

Le cadre présente avantageusement une forme essentiellement rectangulaire. Les barres ou bords 15, 16 sont cotées de canaux creusés 19, tandis que les barres 17, 18 sont pleines.

Le film poreux 13 de base s'étend dans un plan, dans lequel sont situées les premières faces des barres ou bords (15, 16, 17, 18). Le film poreux supérieur 12 recouvre les faces des barres ou bords 17, 18 opposés auxdites premières faces, de manière à former entre les barres 15, 16 une cavité ou canal 20 qui est située sur la face du film 12 opposée à celle tournée vers le cadre 10.

La base de ladite cavité 20 est ainsi formée par le film 12 perméable aux liquides.

L'unité 1 est ainsi adaptée pour former avec l'unité 2 contiguë une chambre ou volume 20 convenant pour recevoir un milieu ou une matrice contenant des cellules. Par exemple, les cellules peuvent être piégées dans une matrice 22 en collagène ou entre deux couches de collagène. Il est évident que d'autres matériaux peuvent convenir pour fixer des cellules. On préfère cependant souvent le collagène. La chambre contient avantageusement deux couches de cellules séparées l'une de l'autre par une couche de collagène.

Le fluide à traiter pénètre dans la chambre ou le passage à travers des canaux 19A, (s'étendant à travers le bord A cause des films poreux 12, 13, le fluide est traité par les cellules contenues dans la matrice 22. Le fluide traité s'écoule hors de la chambre 11 par des canaux 19B (s'étendant à travers le bord 15).

Entre les canaux d'entrée 19A et Les canaux de sortie 19B, la chambre ou passage 11 est dotée de moyens 23 pour diriger l'écoulement du fluide dans la chambre 11. Ces moyens sont par exemple des reliefs portés par le film de base 13. D'autres moyens sont possibles.

Des fibres 24 perméables aux gaz s'étendent à l'intérieur de l'espace ouvert ou cavité 20 et entre les bords 15 et 16. Du gaz contenant de l'oxygène pénètre dans les fibres par des ouvertures ou trous 25 et s'écoule hors de ces fibres par des ouvertures ou trous 26 du bord 15.

Ces fibres s'étendent dans la matrice 22 contenant les cellules, de manière à oxygéner les cellules au cours du traitement.

De préférence on utilise deux membranes contiguës 13A, 13B pour former le film poreux 12, 13. Les membranes 13A tournées vers le canal 11 présentent des pores d'un diamètre d'environ 0,45 µm, tandis que les membranes tournées vers la cavité ou chambre présentent des pores d'un diamètre d'environ 3 µm.

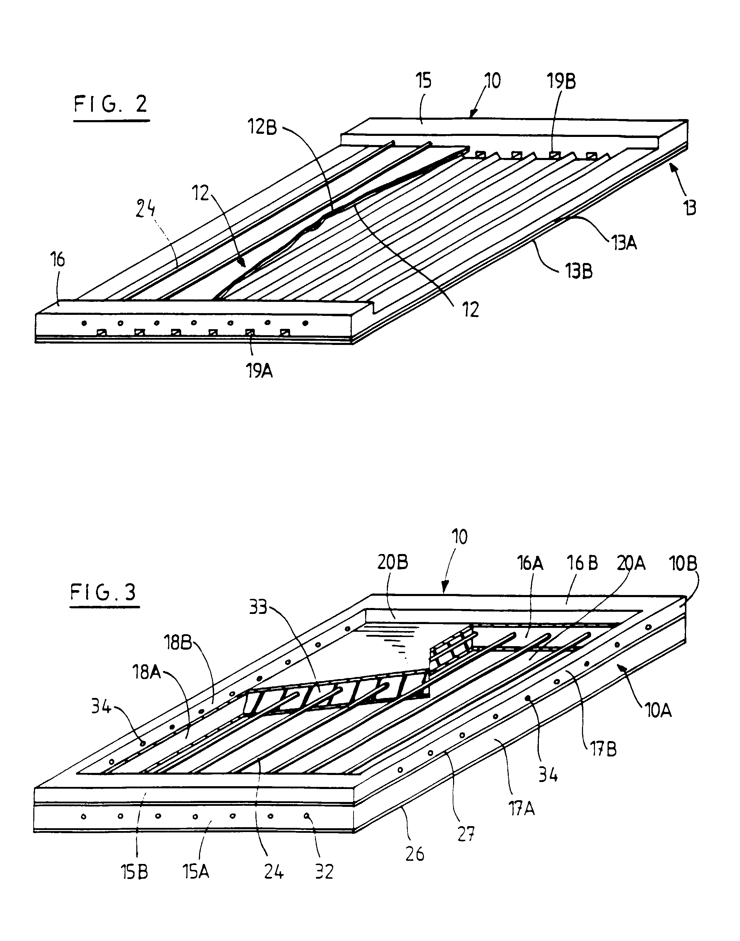

La figure 2 montre une unité du bioréacteur de la figure 1. Ladite unité comporte:
a) un cadre 10;
b) des fibres creuses 24 perméables aux gaz s'étendant entre deux bords opposés 15, 16;
c) des membranes 12A, 12B, 13A, 13B perméables aux liquides (avec un porosité de 0,45 µm ou de 3 µm), entre lesquelles un canal est formé pour l'écoulement du fluide à traiter. Dans cette unité, on n'utilise pas de collagène pour fixer les cellules.

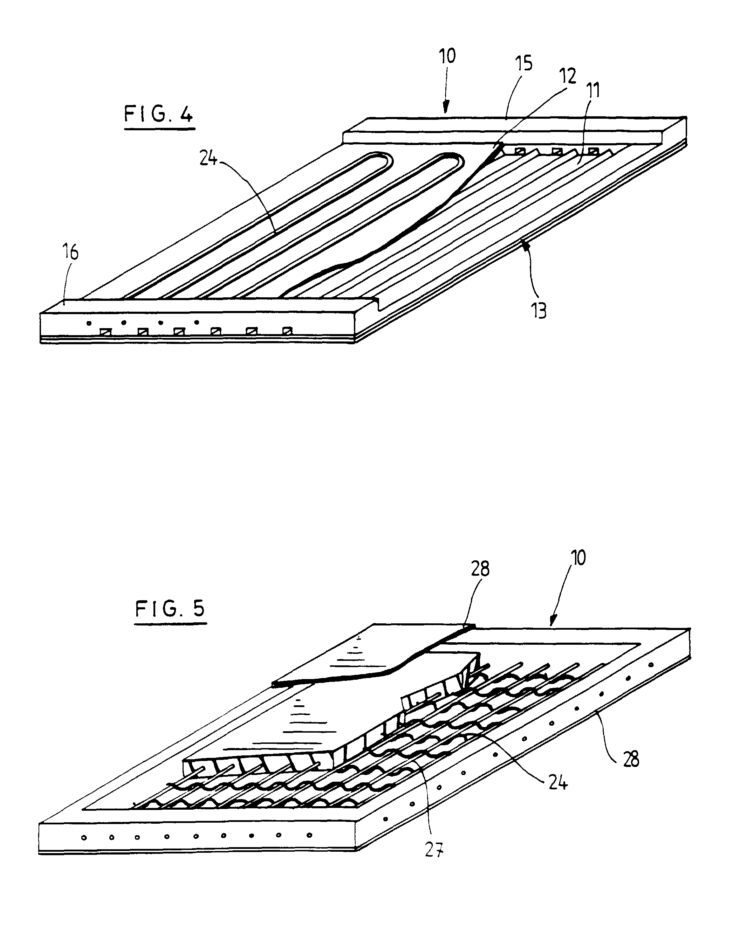

La figure 3 montre un autre mode de réalisation d'une unité convenant pour un bioréacteur selon l'invention.

Ladite unité comporte un cadre 10 constitué de deux éléments 10A, 10B présentant à l'extérieur la forme d'un rectangle et ayant des bords 15A, 16A, 17A, 18A; 15B, 16B, 17B, 18B définissant une cavité rectangulaire 20A, 20B.

Une couche imperméable 26 forme la base de la cavité 20A, tandis qu'une membrane 27 perméable aux liquides située entre les deux éléments 10A, 10B forme la base de la cavité 20B et le couvercle de la cavité 20A.

Entre les bords 15A, 16A s'étendent des fibres creuses 24 perméables aux gaz ainsi que des fibres pleines 32 servant de moyen de renfort.

Les bords 17B, 18B présentent des trous 34 de manière à former des passages d'entrée du fluide à traiter dans la cavité 20B ou de sortie du milieu traité hors de la cavité 20B.

Les fibres creuses 24 sont revêtues d'un matériau, par exemple un matériau contenant du collagène, pour réaliser une surface dans laquelle les cellules sont fixées. Un matériau tel qu'un hydrogel, du PVA, du HEMA, etc. est utilisé pour remplir l'espace entre les fibres de la cavité 20A. Il est cependant avantageux d'appliquer une couche mince de collagène ou d'un matériau similaire au-dessus dudit matériau, pour induire l'adhérence des cellules, ladite couche mince formant la surface plane adaptée à entrer en contact avec la membrane ou film 27 perméable aux liquides. Dans ce cas, la porosité de la membrane ou d'une membrane du film présente par exemple des ocres d'un diamètre inférieur 0,5 µm.

Le cadre 10A, 10B peut être fabriqué en utilisant des composés de moulage. Les fibres creuses sont par exemple alignées longitudinalement à un intervalle constant.

Du composé de moulage est alors appliqué transversalement, aux deux endroits des fibres creuses qui sont distants l'un de l'autre d'une distance correspondant à la longueur du réacteur.

Des extrémités des fibres creuses sont ainsi incorporées dans une matrice, les extrémités libres des fibres creuses étant situées sur une face de la matrice de manière à permettre le passage d'un fluide, de préférence un gaz, à travers les fibres creuses.

La matrice dans laquelle les extrémités libres des fibres creuses sont incorporées peut présenter une épaisseur supérieure à celle des barres reliant ladite matrice. Cependant, dans le mode de réalisation représenté en figure 3, l'épaisseur du cadre 10 reste constante, mais le matériau 33 remplissant l'espace entre les fibres et comportant les fines couches de collagène sur les deux faces ne remplit que partiellement le volume défini par le cadre, de sorte qu'une partie dudit volume est ouverte ou convient pour recevoir un milieu liquide à traiter.

Ladite unité est donc adaptée pour former avec une unité contiguë une chambre ou canal entre la membrane perméable aux liquides d'une première unité et la couche de base de l'unité contiguë, ce canal convenant pour l'écoulement d'un liquide.

La figure 4 montre une unité similaire à celle de la figure 2, à la différence que les fibres creuses (peeméables aux gaz) 24 forment une courbe (forme en U), de sorte que l'entrée du gaz dans les fibres et la sortie du gaz sont situées le long d'un bord commun 16 du cadre 10.

Dans le mode de réalisation de la figure 5, des moyens d'oxygénation sont constitués ce fibres creuses 24 tressées avec les fibres creuses 27 servant au passage du liquide à traiter. Lesdites fibres creuses sont par exemple incorporées dans le matériau tel que de l'hydrogel qui remplit l'espace entre les fibres. Le cadre 10 est avantageusement doté des deux côtés d'une couche imperméable 28.

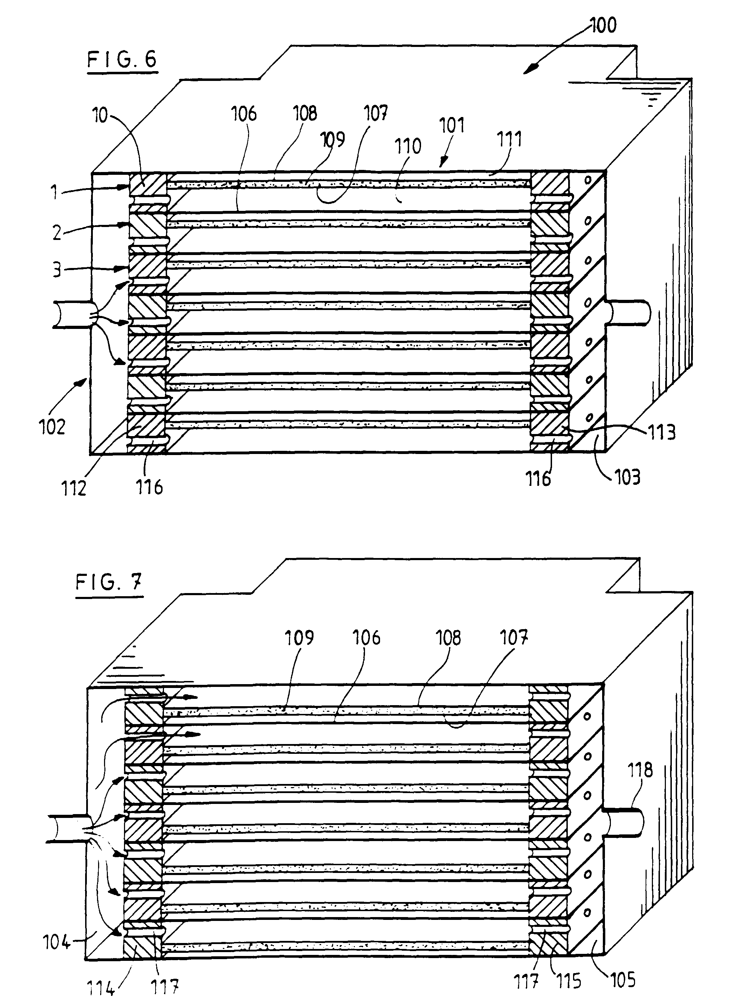

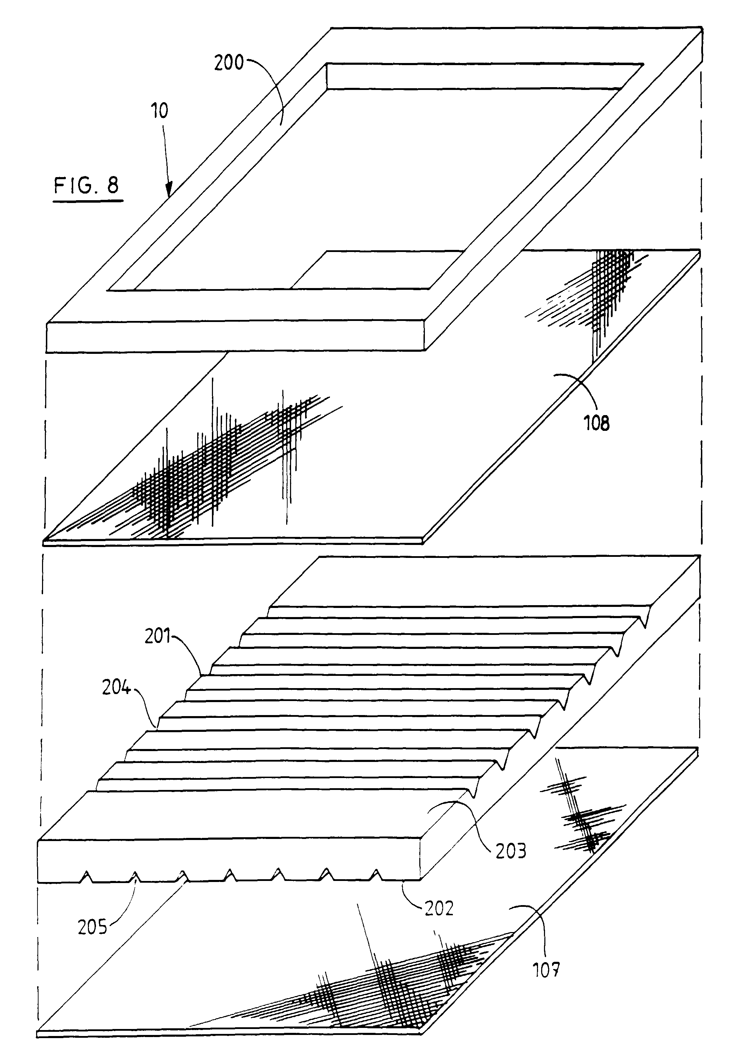

Les figures 6 et 7 sont des vues en coupe transversale d'un système de bioréacteur 100 selon l'invention.

Ledit système de bioréacteur 100 comporte un bioréacteur 101, ledit bioréacteur présentant un collecteur ou distributeur d'entrée 102 pour le liquide s'écoulant dans le bioréacteur, un collecteur de sortie 103 pour le liquide s'écoulant hors du bioréacteur, un collecteur ou distributeur d'entrée 104 pour un gaz, tel que de l'oxygène, s'écoulant dans le bioréacteur et un collecteur de sortie 105 pour le gaz quittant le bioréacteur.

Le bioréacteur 101 comporte plusieurs unités 1, 2, 3, dont chacune est constituée d'un cadre 10 doté d'une couche imperméable de base 106, d'une membrane perméable aux liquides 107 et d'une membrane perméable aux gaz 108.

Entre la membrane perméable aux gaz 108 et la membrane perméable aux liquides 107 est définie une chambre 109 destinée à contenir les cellules, par exemple une matrice contenant des cellules.

Entre la membrane imperméable 106 (destinée à recouvrir le cadre 10 d'une unité contiguë et à séparer deux unités contiguës) et la membrane perméable aux liquides 107, un canal de passage 110 est formé pour le liquide à traiter.

Entre la membrane perméable aux gaz 108 et une membrane ou couche imperméable 106 d'une unité contiguë ou du boîtier du système, un passage ou canal 111 est formé pour le gaz d'oxygénation des cellules.

Le cadre 10 a la forme d'un rectangle et présente quatre bords 112, 113, 114, 115. Les bords opposés 112, 113, qui sont contigus à l'un des collecteurs de liquides 102, 103, sont dotés de trous de passage 116 de sorte que le liquide peut s'écouler du collecteur d'entrée 102 dans le ou les canaux 110 de l'unité du bioréacteur, et peut s'écouler hors dudit canal ou desdits canaux 110 dans le collecteur de sortie 103. Les deux autres bords 114, 115 sont également dotés de trous 117 ou de canaux en creux de manière à permettre le passage de gaz dans le bioréacteur 101 ou hors de celui-ci.

Avantageusement, le collecteur de sortie 103 pour le liquide est doté d'un moyen assurant l'ultra-filtration du liquide après traitement dans le bioréacteur, tandis que sur le tuyau de sortie 118 servant à évacuer ou recycler du gaz provenant du collecteur de sortie de gaz 105, des moyens agissant pour réduire ou augmenter le débit de gaz hors du bioréacteur et donc dans le bioréacteur. De tels moyens sont par exemple une pompe et des vannes assurant une pression spécifique sur une face de la membrane perméable aux gaz, par exemple pour assurer que la pression exercée sur la membrane perméable aux gaz soit essentiellement égale ou inférieure à la pression exercée sur la membrane perméable aux liquides. Par exemple, du gaz est aspiré hors du réacteur. Une telle aspiration s'est avérée être préférable pour la régulation du débit de liquide.

Le bioréacteur de l'invention présente une configuration bien déterminée permettant une activité métabolique élevée. De plus, ce réacteur est facile à fabriquer, et le nombre des unités à utiliser peut être adapté de manière à prendre en compte les besoins du patient.

Le bioréacteur selon l'invention convient pour de nombreux procédés, tels qu'un procédé pour le traitement de cultures de cellules, en particulier d'hépatocytes, sur des fibres creuses ou un film poreux qui sont au moins partiellement perméables aux gaz et/ou aux liquides, de sorte que les cultures de cellules se déposent sur le film ou les fibres creuses, et le milieu de suspension servant de milieu de culture est conduit à travers les fibres creuses ou à travers une chambre.

En médecine et en pharmacie, le bioréacteur peut être utilisé pour effectuer des essais avec des cultures de cellules, et en particulier de cellules du foie (hépatocytes). Cela s'applique par exemple à leur multiplication, leur observation, en particulier l'observation de réaction avec des matières étrangères et/ou des produits toxiques, leur conservation, etc.

En outre, le bioréacteur est une solution au problème de la recherche d'organes de remplacement adéquats.

Le bioréacteur tend également à créer un procédé permettant une culture de masse de manière à obtenir des conditions autant que possible "in vivo", et particulièrement une durée de vie aussi longue que possible.

Dans l'invention, les cultures de cellules sont de préférence incorporées dans une couche de matrice extracellulaire, selon une technique en sandwich connue en soi. Cela assure ainsi une réorganisation du schéma de cellules et une reformation des microvillosités sur le ou les côtés tournés vers les surfaces de la matrice. Cela correspond à la forme naturelle des cellules du foie, et favorise les transferts massiques dans le parcours de membranes sinusoïdales. En plus de ces avantages morphologiques, le maintien du fonctionnement de ces cellules est également essentiel.

La figure 8 montre un autre mode de réalisation d'une unité selon l'invention. Ladite unité comporte un cadre 10 présentant une ouverture centrale 200, une membrane perméable aux gaz 108, un espaceur ou écarteur 201 et une membrane perméable aux liquides 107. Sur ses faces opposées, 202, 203, l'écarteur est doté de sillons 204, 205 de manière à former des canaux pour le passage de gaz (204) et pour le passage de liquides (205). Dans le mode de réalisation représenté, les canaux sont tels que l'écoulement du liquide est perpendiculaire à l'écoulement du gaz.

La membrane perméable aux gaz 108 recouvre le fond du cadre 10 et forme le fond d'une cavité destinée à contenir la cellule ou les cellules pour le traitement.

Selon un mode de réalisation préféré, semblable à celui représenté en figure 8, le plateau 201 est doté de la membrane perméable aux gaz 108 et de la membrane perméable aux liquides 107. Ledit plateau et lesdites membranes forment alors un écarteur destiné à être placé entre deux cadres contigus 200.

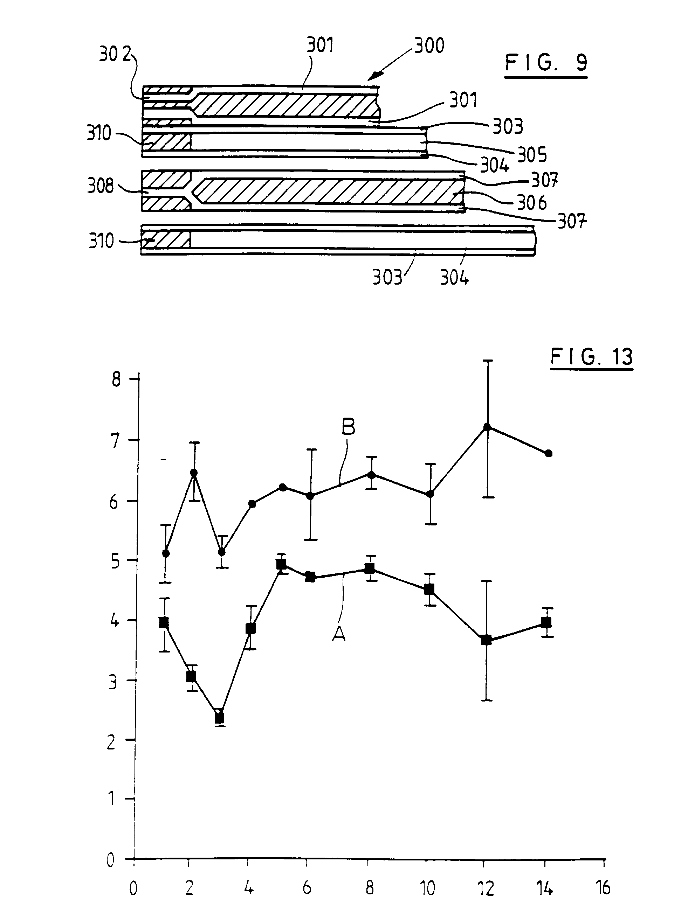

Le figure 9 est une vue partielle éclatée en coupe transversale d'une autre unité.

Cette unité comporte:
- un plateau 300 doté sur ses faces opposées de sillons 301 destinés à former des canaux pour le passage de gaz, et le long d'au moins un des ses bords 302, d'un ou de plusieurs trous de manière à former un canal entre ledit bord et les sillons 301;
- un cadre 310 doté d'une membrane perméable aux gaz 303 et d'une membrane perméable aux liquides 304, de manière à définir entre lesdites membranes une chambre 305 destinée à contenir des cellules pour le traitement;
- un plateau 306 doté sur ses faces opposées de sillons 307, lesdits sillons étant reliés à une entrée 308 et à une sortie pour le liquide à traiter ou traité, et
- un cadre 310 doté d'une membrane perméable aux gaz 303 et d'une membrane perméable aux liquides 304, de manière à définir entre lesdites membranes une chambre 305 destinée à contenir les cellules pour le traitement.

Le plateau 300 est avantageusement doté sur ses deux faces d'une membrane perméable aux gaz 303, tandis que le plateau 308 est doté sur ses deux faces d'une membrane perméable aux liquides 304. Avec leur membrane, lesdits plateaux 300, 308 forment alors un écarteur à placer entre deux cadres contigus 310.

La fixation de la membrane sur les plaques est préférée pour des raisons d'empilement.

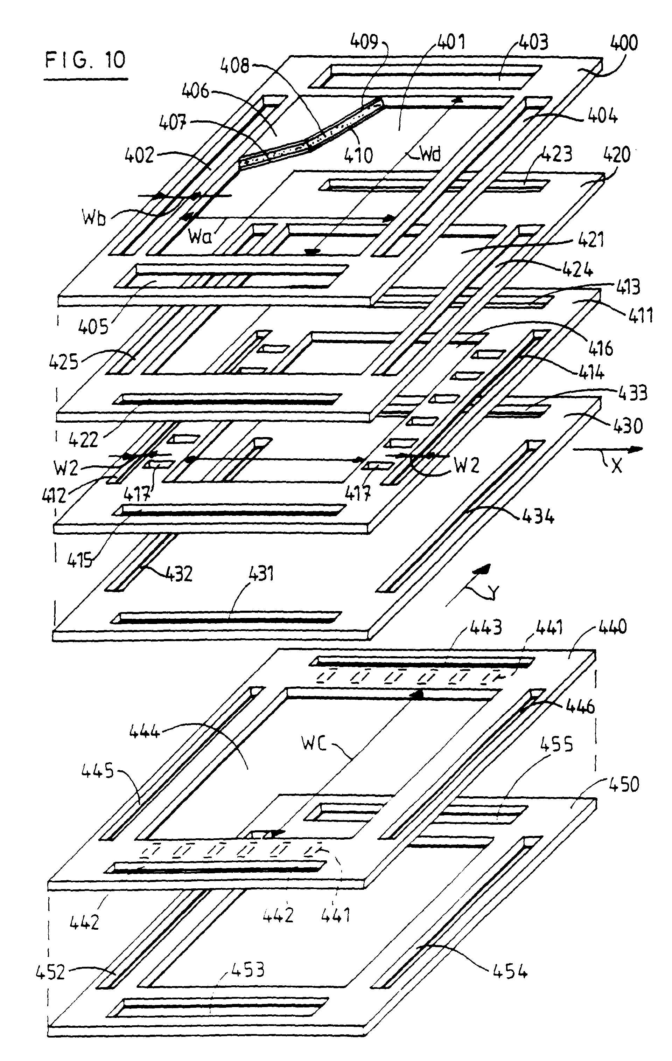

La figure 10 est une vue éclatée d'une unité selon l'invention, ces unités formant un bioréacteur selon l'invention lorsqu'elles sont empilées l'une sur l'autre.

Ladite unité comporte:
- un plateau carré 400 comportant une ouverture centrale 401 et des ouvertures latérales 402, 403, 404, 405 le long des bords du plateau 400, l'ouverture centrale 401 étant dotée d'une membrane perméable aux gaz 406 supérieure, d'un film perméable aux liquides 407 de fond (constitué de deux membranes contiguës) et entre ladite membrane perméable aux gaz supérieure et le film perméable aux liquides du fond, une couche de cellules 408 retenue par des couches de collagène 409, 410;
- un plateau carré 420 comportant une ouverture centrale carrée 421 et des ouvertures latérales 422, 423, 424, 425;
- un plateau carré 411 comportant quatre ouvertures latérales 412, 413, 414, 415 et une ouverture centrale 416, l'ouverture centrale 416 et les ouvertures latérales 412, 414 présentant une largeur W1, W2 inférieure à la largeur Wa, Wb, respectivement de l'ouverture centrale 401 et des ouvertures 402, 404 (largeur dans la direction X); des sillons 417 sont situés entre les ouvertures latérales 412, 414 et l'ouverture centrale 416;
- un plateau carré 430 comportant uniquement quatre ouvertures latétales 431, 432, 433, 434;
- un plateau carré 440 similaire au plateau 411, à la différence que les sillons 441 sont situés entre les ouvertures latérales 442, 443, et que la largeur wc de l'ouverture centrale 444 (largeur dans la direction Y, perpendiculaire à la direction X) est inférieure à la largeur Wd de l'ouverture 401 (dans la direction Y), et
- un plateau carré 450 similaire au plateau 420.

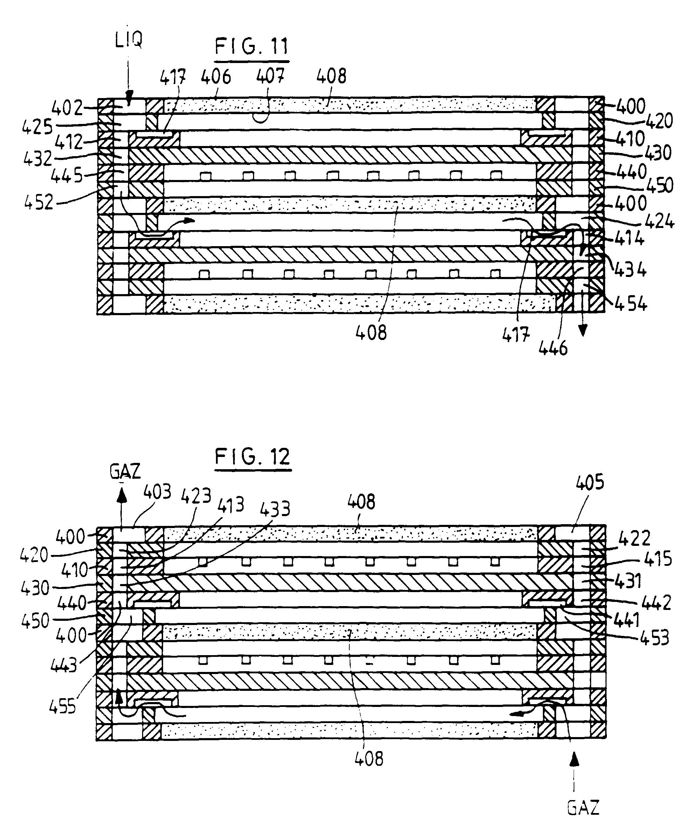

Les ouvertures latérales 402, 412, 425, 432, 445, et 452 forment un canal pour l'écoulement d'entrée du liquide à traiter, tandis que les ouvertures centrales 404, 414, 424, 434, 446 et 454 forment un canal pour l'écoulement de sortie du liquide, après passage devant la membrane perméable aux liquides 410 (c'est à dire à travers les sillons 417 et dans la chambre formée par les ouvertures 415 et 421) (voir figure 11).

Les ouvertures latérales 403, 413, 423, 433, 443 et 455 forment un canal pour l'écoulement de sortie d'un gaz tel que de l'oxygène, tandis que les ouvertures latérales 405, 415, 422, 431, 442 et 453 forment un canal pour l'écoulement d'entrée du gaz.

Le gaz s'écoule devant la membrane perméable aux gaz 406, c'est-à-dire qu'il pénètre et sort de la cavité formée par les ouvertures centrales 451 et 444, à travers le sillons 441 (voir figure 12).

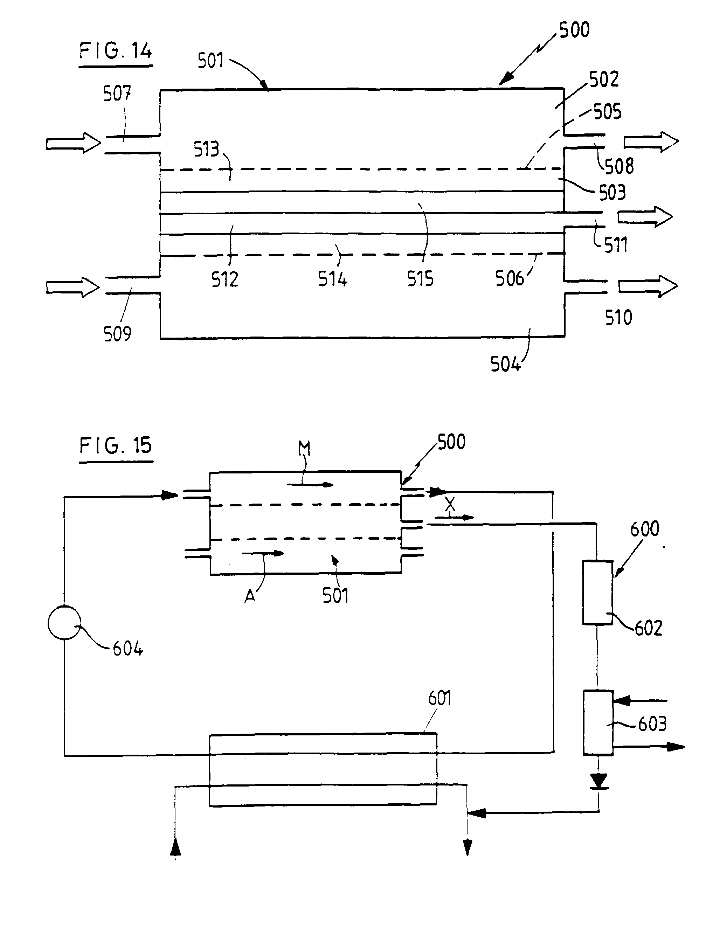

La figure 14 montre un système 500 comportant un bioréacteur 501. Le bioréacteur présente un compartiment pour fluide 502, un compartiment pour cellules 503 et un compartiment pour gaz 504, une membrane perméable aux liquides ou plusieurs membranes perméables aux liquides 505, au moins l'une desdites membranes présentant des pores d'un diamètre inférieur à 0,45 µm, une membrane perméable aux gaz 506, une entrée 507 pour introduire le fluide à traiter, et une sortie 508 pour le fluide s'écoulant hors du compartiment à fluide 502, une entrée 509 et une sortie 510 pour le compartiment à gaz 504, et une sortie 511 pour collecter le fluide s'écoulant hcrs de la chambre ou compartiment à cellules 504.

Le compartiment à cellules 503 comporte deux couches de cellules 512, 513. Le compartiment à cellules 503 comporte dans l'exemple représenté une première couche de collagène 514, contiguë à la membrane perméable aux gaz 506, une couche d'hépatocytes 512, une seconde couche de collagène 515 recouvrant la couche 512, et la couche de cellules de Kupfer 513. Un tel compartiment à cellules convient pour assurer une co-culture.

Comme certains produits biliaires peuvent rester dans le compartiment à cellules 503, il est recommandé d'extraire lesdits produits du compartiment pour avoir un bon fonctionnement, essentiellement constant, des cellules.

La figure 15 montre le système de la figure 14 associé à un moyen de filtration 600 et à un moyen d'immuno-isolation 601. Le moyen de filtration comporte une membrane filtrante 602 empêchant le passage de particules d'un diamètre plus grand que 0,3 µm, et un moyen d'ultrafiltration 603 de manière à extraire la créatinine, l'urée et d'autres composés.

Le moyen d'immuno-isolation est un moyen destiné à isoler du bioréacteur 501 le sang s'écoulant du patient. Un tel moyen d'immuno-isolation comporte par exemple des fibres creuses présentant des propriétés hydrophobes et hydrophiles. Par exemple, 50% des fibres sont des fibres hydrophobes ou lipophiles, tandis que les autres 50% des fibres sont hydrophiles.

Une pompe 604 assure l'écoulement de milieu (M) à travers le bioréacteur 501 et à travers les fibres du moyen d'immuno-isolation 601. De l'air enrichi en oxygène (A) s'écoule à travers le bioréacteur 501.

Le fluide (X1) s'écoulant hors du compartiment à cellules (en deux ou plus de deux couches séparées l'une de l'autre) est filtré et ultrafiltré avant d'être renvoyé dans le sang du patient s'écoulant hors du moyen d'immuno-isolation.

Le débit sanguin dans le moyen d'immuno-isolation est par exemple de 600 ml/minute, tandis que le débit plasmatique dans le moyen d'immuno-isolation et le bioréacteur est de 14 litres/minute.

Le milieu oxygénant peut par exemple être un mélange de 20% O₂ - 80% N₂ pour la phase de fixation des cellules, et un mélange de 10% O₂ - 90% N₂ pour la phase de traitement.

La sortie du fluide hors du compartiment à cellules et le recyclage dans le sang du patient dudit fluide après filtration permettent le transfert d'hémoglobine modifiée (poids moléculaire de 4000-5000) dans le sang.

L'efficacité du bioréacteur de l'invention ressort de la figure 13.

En effet, pour des hépatocytes primaires placés sur un disque en plastique (courbe A), la sécrétion d'albumine varie d'environ 2 à 5 µg/heure/10° cellules (2 au jour 3 et environ 4 au jour 14) , tandis que la fixation d'albumine (courbe B) d'hépatocytes primaires dans le bioréacteur de l'invention (une membrane perméable aux gaz étant utilisée pour le transfert des gaz) varie d'environ 5 à 7 (5 au jour 3 et environ 7 au jour 14) µg/heure/10⁶ cellules.

La courbe B montre que le mode de conception du bioréacteur permet une oxygénation adéquate à des pressions partielles physiologiques d'oxygène et indique le rendement accru du transfert d'oxygène, avec pour résultat une fonction de synthèse accrue des hépatocytes. Ces pressions partielles ambiantes d'oxygène sont inférieures à celles habituellement utilisées dans des cultures standard sur des supports imperméables (20% oxygène, cultures sur des plateaux en plastique standard pour cultures de tissus, revêtus de collagène).

En utilisant le bioréacteur de l'invention, on obtient une sécrétion c'est-à-dire un travail, beaucoup plus constant du bioréacteur, la variation étant inférieure à 1 µg/heure/10⁶ cellules par rapport à la valeur moyenne (environ 6), c'est-à-dire une variation d'environ 15% ou moins.

Lorsque l'on utilise des disques en plastique sans membrane perméable aux gaz, la variation est d'environ 1,5 µg/heure/10° cellules par rapport à la valeur moyenne (environ 3,5), c'est-à-dire une variation maximale possible d'environ 40% par rapport à la valeur moyenne.

Il faut faire remarquer que la conception du bioréacteur de l'invention permet une distribution et une fourniture uniformes d'oxygène aux cellules pendant la phase de fixation. Normalement, la circulation du milieu de culture devrait être interrompue pour permettre le dépôt des cellules. Cependant, pendant la phase de fixation, les cellules présentent une demande accrue en oxygène. On a observé dans des études une efficacité accrue de la fixation sur des membranes perméables aux gaz. Interrompre la circulation du liquide comme dans les réacteurs standard est désavantageux, en particulier pour des cellules primaires.

Le bioréacteur de l'invention convient pour des cellules recouvertes par du collagène, mais également pour des cellules non recouvertes par du collagène (par exemple cellules hématopoïétiques, en réalité, en dehors des hépatocytes, la plupart des autres cellules ne nécessitent pas une fixation bipolaire au collagène).

Des membranes perméables aux liquides sont également perméables aux gaz. Il peut exister des situations qui ne nécessitent pas une circulation spécifique de liquide, particulièrement en ce qui concerne des cellules qui sont moins sensibles aux forces de cisaillement que les hépatocytes. Dans un tel cas, un milieu de culture peut également s'écouler dans la chambre contenant les cellules. Dans ce cas, une membrane perméable exclusivement aux gaz pourrait être située des deux côtés du compartiment à cellules, pour permettre la fixation des cellules des deux côtés. Le compartiment à cellules nécessite alors une entrée et une sortie de manière à avoir une circulation de liquide à travers le compartiment.

Le cadre séparant la membrane perméable aux gaz et la membrane perméable aux liquides présente avantageusement une ouverture de remplissage en cellules.

Les canaux de l'écarteur ou de la plaque peuvent être creusés, imprimés ou appliqués à l'aide d'un masque.

## Revendications

1. Bioréacteur pour un traitement par cellules d'un milieu, ledit bioréacteur comportant :
(a) un premier canal (11) pour l'écoulement dudit milieu à traiter ;
(b) un second canal (24) pour l'écoulement d'un gaz contenant de l'oxygène ;
(c) un élément définissant une chambre (20) contenant au moins deux couches de cellules pour traiter le milieu, lesdites couches étant séparées l'une de l'autre par une couche de collagène ;
(d) une membrane perméable au gaz (24) séparant ladite chambre du second canal dans lequel s'écoule le gaz contenant de l'oxygène, et
(e) un film séparant ladite chambre (20) dudit premier canal (11), ledit film étant constitué de deux membranes perméables aux liquides contiguës, une première membrane (13B) présentant des pores ou des ouvertures de diamètre supérieur à 3 µm ou d'environ 3 µm, tandis que la deuxième membrane (13A) présente des pores ou des ouvertures de diamètre inférieur à 3 µm.

2. Bioréacteur suivant la revendication 1, caractérisé en ce que ledit film est constitué de deux membranes perméables aux liquides contiguës, une première membrane (13B) présentant des pores ou des ouvertures de diamètre supérieur à 3 µm ou d'environ 3 µm, tandis que la deuxième membrane présente des pores ou des ouvertures de diamètre inférieur à 0,5 µm,

3. Bioréacteur suivant la revendication 1, caractérisé en ce que ledit film est constitué de deux membranes perméables aux liquides contiguës, une première membrane présentant des pores ou des ouvertures de diamètre supérieur à 3 µm, tandis que la deuxième membrane présente des pores ou des ouvertures de diamètre inférieur à 0,3 µm.

4. Bioréacteur suivant l'une des revendications 1 à 3, caractérisé en ce que la membrane présentant des pores ou des ouvertures de diamètre supérieur à µm ou d'environ 3 µm est tournée vers la chambre, tandis que la deuxième membrane présentant des pores ou des ouvertures de diamètre inférieur à 3 µm est tournée vers le premier canal.

5. Bioréacteur selon l'une quelconque des revendications 1 à 4, caractérisé en ce que les membranes perméables aux liquides sont des membranes présentant des caractéristiques hydrophobes ou une combinaison de caractéristiques hydrophobes et hydrophiles.

6. Bioréacteur selon l'une des revendications 1 à 5, caractérisé en ce que la chambre est dotée d'une sortie de manière à extraire le milieu passant à travers les membranes perméables aux liquides en provenance du canal dans lequel s'écoule le milieu à traiter.

7. Bioréacteur selon l'une quelconque des revendications 1 à 6, caractérisé en ce qu'une couche de cellules contient au moins une cellule ou des cellules non présentes dans l'autre couche de cellules, ou en ce qu'une couche de cellules contient une quantité plus élevée d'une ou de plusieurs cellules spécifiques que l'autre couche.

8. Bioréacteur selon l'une quelconque des revendications précédentes, caractérisé en ce que la chambre comporte une entrée et une sortie de manière à permettre un écoulement du milieu cellulaire.

9. Bioréacteur selon l'une quelconque des revendications précédentes, caractérisé en ce qu'il comporte plusieurs unités empilées constituées chacune d'un cadre doté de deux couches, de manière à définir entre elles un canal au passage pour l'écoulement d'un milieu liquide ou d'un gaz, une desdites couches comportant des membranes perméables aux liquides ou une membrane perméable aux gaz.

10. Bioréacteur selon la revendication 9, caractérisé en ce que le cadre présente des bords définissant entre eux une cavité ou un passage, une face de ladite cavité ou passage étant constituée d'une des couches, ladite cavité ou passage contenant les cellules pour le traitement.

11. Bioréacteur selon la revendication 10, caractérisé en ce que l'unité comporte un cadre ouvert définissant une cavité ou passage destinée à contenir les cellules pour le traitement, plusieurs membranes perméables aux liquides couvrant ledit cadre ou formant le fond du cadre, une membrane perméable aux gaz formant le fond du cadre ou recouvrant ledit cadre, une feuille ou couche présentant au moins un canal pour l'écoulement d'un liquide contigu à une feuille ou couche perméable aux liquides, avec au moins un canal pour l'écoulement d'un gaz, contigu à la membrane perméable aux gaz.

12. Bioréacteur selon la revendication 11, caractérisé en ce qu'une feuille ou couche est dotée sur une de ses faces d'un canal ou de canaux pour l'écoulement de liquides, contigus à une membrane perméable aux liquides, et sur sa face opposée est dotée d'un canal ou de canaux pour l'écoulement d'un gaz, contigus à une membrane perméable aux gaz.

13. Bioréacteur selon la revendication 11 ou 12, caractérisé en ce que ladite feuille ou couche est gravée de manière à définir des canaux gravés sur une de ses faces.

14. Bioréacteur selon la revendication 10, caractérisé en ce que le cadre est doté d'une couche de fond"qui n'est pas perméable et une couche supérieure qui est perméable aux liquides, lesdites couches couvrant des faces du cadre de manière à définir entre lesdites couches un canal pour l'écoulement d'un liquide, et en ce que la cadre est doté d'au moins une entrée et d'au moins une sortie formant à travers le cadre des passages vers le canal d'écoulement d'un liquide.

15. Bioréacteur selon la revendication 10, caractérisé en ce que le cadre comporte des bords à canaux gravés, lesdits bords étant reliés l'un à l'autre au moyen d'un bord non gravé présentant une épaisseur moindre que les bords gravés; une couche constituée de préférence d'une membrane perméable s'étendant entre les bords gravés et recouvrant au moins partiellement les bords non gravés, de manière à définir une cavité ou espace ouvert sur la face de ladite couche tournée vers la face recouvrant les bords non gravés.

16. Bioréacteur selon la revendication 15, caractérisé en ce qu'il est doté de moyens s'étendant à travers ou dans la cavité ou l'espace ouvert, et qui sont perméables aux gaz de manière à assurer une oxygénation de la cavité ou espace ouvert.

17. Bioréacteur selon l'une des revendications 1 à 16, caractérisé en ce qu'il comporte plusieurs unités qui sont empilées, chaque unité comportant un cadre définissant une cavité ou un milieu contenant les cellules pour le traitement, une membrane perméable aux liquides, un écarteur doté sur ses deux faces de canaux gravés et une membrane perméable aux gaz.

18. Bioréacteur selon l'une quelconque des revendications 1 à 17, dans lequel les cellules de chacune des couches de cellules séparées l'une de l'autre sont incorporées dans une matrice contenant du collagène ou sont piégées entre deux couches de collagène.

19. Système comportant un bioréacteur selon l'une quelconque des revendications 1 à 18, ledit bioréacteur présentant une entrée pour introduire le milieu à traiter dans le canal à liquide, une sortie pour le milieu s'écoulant hors dudit canal à liquide, une entrée d'écoulement de gaz dans le bioréacteur, une sortie pour le gaz quittant le réacteur et une sortie pour collecter le liquide s'écoulant hors de la chambre, le système étant en outre doté de moyens de filtration du liquide s'écoulant hors de la chambre.

20. Système selon la revendication 19, caractérisé en ce qu'il comporte des moyens d'ultrafiltration du liquide s'écoulant hors de la chambre ou est associé à de tels moyens.

21. Système selon la revendication 19 ou 20, dans lequel le bioréacteur est associé à un dispositif d'immuno-isolation.

22. Système selon la revendication 21, dans lequel le dispositif d'immuno-isolation comporte des membranes perméables pour séparer l'écoulement du sang dans le dispositif et l'écoulement du milieu à traiter dans le bioréacteur et s'écoulant hors du bioréacteur.

23. Procédé de culture de cellules, caractérisé par le fait qu'on utilise un bioréacteur selon l'une quelconque des revendications 1 à 18.

24. Support hépatique comportant un bioréacteur selon l'une quelconque des revendications 1 à 18, de préférence un système selon l'une quelconque des revendications 19 à 23.

25. Procédé de différentiation de cellules, caractérisé par le fait qu'on utilise un bioréacteur selon l'une quelconque des revendications 1 à 18.

## Patentansprüche

1. Bioreaktor zur Behandlung eines Mediums mit Zellen, welcher folgendes aufweist:
a) einen ersten Kanal (11) für den Fluß des zu behandelnden Mediums;
b) einen zweiten Kanal (24) für das Durchströmen eines Sauerstoff enthaltenden Gases;
c) ein Element, welches eine Kammer (20) definiert, die mindestens zwei Zellschichten zur Behandlung des Mediums enthält, welche durch eine Kollagenschicht voneinander getrennt sind;
d) eine gasdurchlässige Membran (24), welche die Kammer vom zweiten Kanal trennt, in welchem das Sauerstoff enthaltende Gas strömt, und
e) einen Film, welcher die Kammer (20) von dem ersten Kanal (11) trennt, wobei dieser Film aus zwei aneinandergrenzenden flüssigkeitsdurchlässigen Membranen gebildet ist, von denen die erste (13B) Poren bzw. Öffnungen mit einem Durchmesser von mehr als 3 µm oder rund 3 µm aufweist, wohingegen die zweite Membran (13A) Poren bzw. Öffnungen mit einem Durchmesser von weniger als 3 µm aufweist

2. Bioreaktor nach Anspruch 1, dadurch gekennzeichnet, daß der Film aus zwei aneinander anschließenden flüssigkeitsdurchlässigen Membranen besteht, wobei die erste Membran (13B) Poren bzw. Öffnungen mit einem Durchmesser von mehr als 3 µm oder rund 3 µm aufweist, wohingegen die zweite Membran Poren bzw. Öffnungen mit einem Durchmesser von weniger als 0,5 µm aufweist.

3. Bioreaktor nach Anspruch 1, dadurch gekennzeichnet, daß der Film aus zwei aneinander anschließenden flüssigkeitsdurchlässigen Membranen besteht, wobei die erste Membran Poren bzw. Öffnungen mit einem Durchmesser von mehr als 3 µm oder rund 3 µm aufweist, wohingegen die zweite Membran Poren bzw. Öffnungen mit einem Durchmesser von weniger als 0,3 µm aufweist.

4. Bioreaktor nach einem der Ansprüche 1 bis 3, dadurch gekennzeichnet, daß die Membran, die Poren bzw. Öffnungen mit einem Durchmesser von mehr als 3 µm aufweist, der Kammer zugewandt ist, wohingegen die zweite Membran mit Poren bzw. Öffnungen mit einem Durchmesser von weniger als 0,3 µm dem ersten Kanal zugewandt ist.

5. Bioreaktor nach einem der Ansprüche 1 bis 4, dadurch gekennzeichnet, daß die flüssigkeitsdurchlässigen Membranen jeweils Membranen mit hydrophoben Eigenschaften oder mit hydrophoben Eigenschaften in Verbindung mit hydrophilen Eigenschaften sind.

6. Bioreaktor nach einem der Ansprüche 1 bis 5, dadurch gekennzeichnet, daß die Kammer einen Auslaß in der Form aufweist, daß das aus dem Kanal, in dem das zu behandelnde Medium fließt, durch die flüssigkeitsdurchlässigen Membranen fließende Medium abgezogen wird.

7. Bioreaktor nach einem der Ansprüche 1 bis 6, dadurch gekennzeichnet, daß eine Schicht Zellen mindestens eine Zelle bzw. Zellen enthält, welche nicht in der anderen Schicht Zellen vorhanden sind, bzw. daß Schicht Zellen eine oder mehrere spezifische Zellen in einer Menge enthält, die größer ist als die Menge in der anderen Schicht.

8. Bioreaktor nach einem der vorhergehenden Ansprüche, dadurch gekennzeichnet, daß die Kammer einen Einlaß und einen Auslaß in der Form aufweist, daß eine Strömung des Zellenmediums möglich ist.

9. Bioreaktor nach einem der vorhergehenden Ansprüche, dadurch gekennzeichnet, daß er mehrere übereinander gestapelte Einheiten aufweist, die jeweils aus einem Rahmen bestehen, der mit zwei Schichten in der Weise versehen ist, daß zwischen diesen ein Durchlaßkanal für die Strömung eines flüssigen Mediums oder eines Gases definiert ist, wobei eine der Schichten flüssigkeitsdurchlässige Membranen oder eine gasdurchlässige Membran aufweist.

10. Bioreaktor nach Anspruch 9, dadurch gekennzeichnet, daß der Rahmen Kanten aufweist, welche zwischen sich einen Hohlraum bzw. einen Durchlaß definieren, wobei eine Seite des Hohlraums bzw. Durchlasses von einer der Schichten gebildet wird und wobei der Hohlraum bzw. Durchlaß die Zellen für die Behandlung enthält.

11. Bioreaktor nach Anspruch 10, dadurch gekennzeichnet, daß die Einheit einen offenen Rahmen aufweist, welcher einen Hohlraum bzw. einen Durchlaß definiert, der zur Aufnahme der Zellen für die Behandlung bestimmt ist, wobei mehrere flüssigkeitsdurchlässige Membranen den Rahmen bedecken oder den Boden des Rahmens bilden, wobei eine gasdurchlässige Membran den Boden des Rahmens bildet bzw. den Rahmen bedeckt, wobei eine Lage bzw. Schicht mindestens einen Kanal für die Strömung einer Flüssigkeit direkt neben an einer flüssigkeitsdurchlässigen Lage bzw. Schicht mit mindestens einem Kanal für die Strömung eines Gases direkt neben der gasdurchlässigen Membran aufweist.

12. Bioreaktor nach Anspruch 11, dadurch gekennzeichnet, daß eine Lage bzw. Schicht auf einer ihrer Seiten mit einem Kanal bzw. mit Kanälen für die Strömung von Flüssigkeiten direkt neben einer flüssigkeitsdurchlässigen Membran aufweist, und auf der gegenüberliegenden Seite mit einem Kanal bzw. Kanälen für die Strömung eines Gases direkt neben einer gasdurchlässigen Membran versehen ist.

13. Bioreaktor nach Anspruch 11 oder 12, dadurch gekennzeichnet, daß die Lage bzw. Schicht in der Weise graviert ist, daß Kanäle definiert sind, die auf einer ihrer Seiten eingelassen sind.

14. Bioreaktor nach Anspruch 10, dadurch gekennzeichnet, daß der Rahmen mit einer undurchlässigen unteren Schicht und einer flüssigkeitsdurchlässigen oberen Schicht versehen ist, wobei die Schichten die Flächen des Rahmens in der Weise bedecken, daß zwischen den Schichten ein Kanal für die Strömung einer Flüssigkeit definiert ist, und daß der Rahmen unter Bildung von Durchlässen durch den Rahmen zum Strömungskanal einer Flüssigkeit hin mit mindestens einem Einlaß und mindestens einem Auslaß versehen ist.

15. Bioreaktor nach Anspruch 10, dadurch gekennzeichnet, daß der Rahmen Kanten mit eingravierten Kanälen aufweist, wobei die Kanten mit Hilfe einer nicht gravierten Kanten untereinander verbunden sind, welche eine geringere Dicke als die gravierten Kanten aufweist; ferner eine vorzugsweise aus einer durchlässigen Membran bestehende Schicht, welche sich zwischen den gravierten Kanten in der Weise erstreckt und die nicht gravierten Kanten zumindest teilweise bedeckt, daß ein Hohlraum bzw. ein offener freier Raum auf der Seite der Schicht definiert ist, die der Fläche, welche die nicht gravierten Kanten bedeckt, zugewandt ist.

16. Bioreaktor nach Anspruch 15, dadurch gekennzeichnet, daß er Einrichtungen aufweist, die sich durch den Hohlraum bzw. den offenen freien Raum hindurch bzw. in diesem erstrecken, und die in der Weise gasdurchlässig sind, daß eine Sauerstoffanreicherung des Hohlraums bzw. des offenen freien Raumes gewährleistet ist.

17. Bioreaktor nach einem der Ansprüche 1 bis 16, dadurch gekennzeichnet, daß er mehrere übereinander gestapelte Einheiten aufweist, von denen jede einen Rahmen aufweist, der einen Hohlraum definiert, oder ein die Zellen für die Behandlung enthaltendes Medium, ferner eine flüssigkeitsdurchlässige Membran, einen Abstandhalter, der auf beiden Seiten mit eingravierten Kanälen versehen ist, und eine gasdurchlässige Membran.

18. Bioreaktor nach einem der Ansprüche 1 bis 17, bei welchem die Zellen in jeder der voneinander getrennten Zellenschichten in eine Matrix einbezogen sind, welche Kollagen enthält, oder zwischen zwei Kollagenschichten eingeschlossen sind.

19. System mit einem Bioreaktor nach einem der Ansprüche 1 bis 18, wobei der Bioreaktor einen Einlaß zur Einleitung des zu behandelnden Mediums in den Flüssigkeitskanal, einen Auslaß für das aus dem Flüssigkeitskanal ausströmende Medium, einen Einlaß für das Einströmen von Gas in den Bioreaktor, einen Auslaß für das aus dem Reaktor austretende Gas, und einen Auslaß zum Auffangen der aus der Kammer ausfließenden Flüssigkeit aufweist, wobei das System außerdem mit Einrichtungen zum Filtern der aus der Kammer ausfließenden Flüssigkeit ausgestattet ist.

20. System nach Anspruch 19, dadurch gekennzeichnet, daß es Einrichtungen zur Ultrafiltrierung der aus der Kammer ausfließenden Flüssigkeit aufweist oder an eine derartige Einrichtung angeschlossen ist.

21. System nach Anspruch 19 oder 20, bei welchem der Bioreaktor an eine Einrichtung zur Immunisolierung angeschlossen ist.

22. System nach Anspruch 21, bei welchem die Einrichtung zur Immunisolierung durchlässige Membranen zum Trennen des Blutstroms in der Vorrichtung vom Strom des in dem Bioreaktor zu behandelnden Mediums, das aus dem Bioreaktor ausfließt, aufweist.

23. Verfahren zur Zellkultur, dadurch gekennzeichnet, daß ein Bioreaktor nach einem der Ansprüche 1 bis 18 eingesetzt wird.

24. Leberträger, welcher einen Bioreaktor nach einem der Ansprüche 1 bis 18 aufweist, vorzugsweise ein System nach einem der Ansprüche 19 bis 23.

25. Verfahren zur Differenzierung von Zellen, dadurch gekennzeichnet, daß ein Bioreaktor nach einem der Ansprüche 1 bis 18 eingesetzt wird.

## Claims

1. Bioreactor for a cell treatment of a medium, said bioreactor comprising :
(a) a first channel (11) for the flow of medium to be treated ;
(b) a second channel (24) for the flow an oxygen containing gas ;
(c) an element defining a chamber (20) containing at least two layers of cells for treating the medium, the said layers being separated the one from the other by means of a collagen layer,
(d) a gas permeable membrane (24) separating the said chamber from the second channel in which the oxygen containing gas flows, and
(e) a film separating the said chamber (20) from said first channel (11), the said film comprising two adjacent liquid permeable membranes, a first membrane (13B) having pores or openings with a diameter greater than 3 µm or equal to about 3 µm, while the second membrane (13A) has pores or openings with a diameter lower than 3 µm.

2. Bioreactor according to claim 1 characterized in that the said film comprises two adjacent liquid permeable membranes, a first membrane (13B) having pores or openings with a diameter greater than 3 µm or equal to about 3 µm, while the second membrane has pores or openings with a diameter lower than 0.5 µm.

3. Bioreactor according to claim 1, characterized in that the said film comprises two adjacent liquid permeable membranes, a first membrane having pores or openings with a diameter greater than 3 µm, while the second membrane has pores or openings with a diameter lower than 0.3 µm.

4. Bioreactor according to anyone of the claims 1 to 3, characterized in that the membrane having pores or openings with a diameter greater than 3 µm or equal to about 3 µm is directed towards the chamber, while the second membrane having pores or openings with a diameter lower than 3 µm is directed towards the first channel.

5. Bioreactor according to anyone of the claims 1 to 4, characterized in that the liquid permeable membranes are membranes with hydrophobic characteristics or with a combination of hydrophobic and hydrophilic characteristics.

6. Bioreactor according to anyone of the claims 1 to 5, characterized in that the chamber is provided with an outlet for removing medium issuing through the liquid permeable membranes from the channel in which the medium to be treated flows.

7. Bioreactor according to anyone of the claims 1 to 6, characterized in that a cells layer contains at least a cell or cells not present in the other cells layer, or in that a cells layer contains a higher amount of a specific cell or of several specific cells than the other layer.

8. Bioreactor according to anyone of the preceding claims, characterized in that the chamber comprises an inlet and an outlet so that a flow of cell medium is possible.

9. Bioreactor according to anyone of the preceding claims, characterized in that it comprises a plurality of stacked units consisting each of a frame provided with two layers so as to define therebetween a channel or passage for the flow of a liquid medium or of a gas, one of said layers comprising liquid permeable membranes or a gas permeable membrane.

10. Bioreactor according to claim 9, characterized in that the frame has edges defining therebetween a cavity or passage, one face of said cavity or passage being one of said layers, said cavity or passage containing the cells for the treatment.

11. Bioreactor according to claim 10, characterized in that the unit comprises an open frame defining a cavity or passage for containing the cells for the treatment, several liquid permeable membranes covering the said frame or forming the bottom of the frame, a gas permeable membrane forming the bottom of the frame or covering the said frame, a sheet or layer with at least one channel for the flow of a liquid adjacent to a sheet or layer permeable to liquids, with at least one channel for the flow of gas adjacent to the gas permeable membrane.

12. Bioreactor according to claim 11, characterized in that a sheet or layer is provided on one of its faces with channel or channels for the flow of liquids adjacent to a liquid permeable membrane and on its opposite face with channel or channels for the flow of gas adjacent to a gas permeable membrane.

13. Bioreactor according to claim 11 or 12, characterized in that the said sheet or layer is engraved so as to define on one of its faces engraved channels.

14. Bioreactor according to claim 10, characterized in that the frame is provided with a bottom layer which is not permeable and with an upper layer which is liquid permeable, said layers covering faces of the frame so as to define between said layers a channel for the flow of a liquid, and in that the frame is provided of at least one inlet and at least one outlet forming through the frame passages towards the channel for the flow of a liquid.

15. Bioreactor according to claim 10, characterized in that the frame comprises edges with engraved channels, said edges being linked the one to another by means of a not engraved edge having a thinner thickness than the engraved edges ; a layer, preferably a permeable membrane, extending between the engraved edges and covering at least partly the not engraved edges so as to define a cavity or open space on the face of the said layer directed towards the face covering the not engraved edges.

16. Bioreactor according to claim 15, characterized in that it is provided with means extending through or within the cavity or open space and which are gas permeable so as to ensure an oxygenation in the cavity or open space.

17. Bioreactor according to anyone of the claims 1 to 16, characterized in that it comprises a plurality of units which are stacked, each unit comprising a frame defining a cavity or a medium containing the cells for the treatment, a liquid permeable membrane, a spacer provided on its both faces with engraved channels and a gas permeable membrane.

18. Bioreactor according to anyone of the claims 1 to 17, characterized in that the cells of the cell layers separated the one from the other are embedded into a matrix containing collagen or are entrapped between two collagen layers.

19. System comprising a bioreactor according to anyone of the claims 1 to 18, the said bioreactor having an inlet for introducing the medium to be treated in the liquid channel, an outlet for the medium flowing out of said liquid channel, an inlet for the flow of gas into the bioreactor, an outlet for gas flowing out of the bioreactor, and an outlet for collecting liquid flowing out of the chamber, the system being further provided with filtration means of the liquid flowing out of the chamber.

20. System according to claim 19, characterized in that it comprises means for ultrafiltrating the liquid flowing out of the chamber or it is associated with such means.

21. System according to claim 19 or 20, in which the bioreactor is associated with an immunoisolation device.

22. System according to claim 21, in which the immunoisolation device comprises permeable membranes for separating the blood flow in the device and the flow of medium to be treated in the bioreactor and flowing out of said bioreactor.

23. Process for the culture of cells, characterized in that a bioreactor according to anyone of the claims 1 to 18 is used.

24. Liver support comprising a bioreactor according to anyone of the claims 1 to 18, preferably a system according to anyone of the claims 19 to 23.

25. Process for cells differentiation, characterized in that a bioreactor according to anyone of the claims 1 to 18 is used.
